# EUROPÄISCHE PATENTSCHRIFT

(11) **EP 2 921 124 B1**
(45) Veröffentlichungstag und Bekanntmachung des Hinweises auf die Patenterteilung: **30.09.2020**
(21) Anmeldenummer: 15159204.5
(22) Anmeldetag: 16.03.2015
(51) Int. Cl.: A61B 17/70

(54) **Gabelförmiges Stabeinsetzinstrument**
FORKED ROD INSERTION INSTRUMENT
INSTRUMENT D'INSERTION DE TIGE EN FORME DE FOURCHE

(30) Priorität: 20.03.2014 DE 102014103874
(43) Veröffentlichungstag der Anmeldung: 23.09.2015
(73) Patentinhaber: Aesculap AG, 78532 Tuttlingen (DE)
(72) Erfinder: Lindner, Stephan, 78573 Wurmlingen (DE)
(74) Vertreter: Winter, Brandl, Fürniss, Hübner, Röss, Kaiser, Polte - Partnerschaft mbB

(56) Entgegenhaltungen:
- US-A1- 2005 192 589
- US-A1- 2008 125 788
- US-A1- 2013 041 415
- US-A1- 2013 261 680

## Beschreibung

### Technisches Gebiet

Die vorliegende Erfindung bezieht sich auf ein Stabeinsetzinstrument gemäß dem Oberbegriff des Anspruchs 1, insbesondere auf einen Stabeinsetzer mit dessen Hilfe ein Verbindungsstab in Tulpen benachbarter Pedikelschrauben im Rahmen einer dorsalen Wirbelsäulenstabilisierung eingesetzt werden kann, sowie ein Stabeinsetzset gemäß einem nebengeordneten Anspruch.

### Stand der Technik

Um bei Wirbelsäulenstabilisierungen Verbindungsstäbe in Tulpen benachbarter Pedikelschrauben einzusetzen, sind bereits viele verschiedene Ausgestaltungen von Stabeinsetzern entwickelt worden. Beispielhaft seien hier nur die Druckschriften WO 2011/143550 A1, US 2011/0313475 A1 und EP 2 305 154 A1 genannt.

Ein Problem bei bisher bekannten Stabeinsetzern besteht zum einen darin, dass bei diesen ein einzusetzender Verbindungsstab und der entsprechende Greifer des Stabeinsetzers im Wesentlichen in einer Ebene liegen. Will man die Beschädigung von Gewebe, das die Pedikelschrauben umgibt, möglichst gering halten, beziehungsweise will man den für den operativen Eingriff nötigen Einschnitt möglichst klein halten, wäre es von Vorteil, wenn der Verbindungsstab zunächst im Wesentlichen senkrecht in den Körper und dann in einer Schwenkbewebung in die Tulpen benachbarter Pedikelschrauben eingeführt werden könnte. Da vor dem Einsetzen des Verbindungsstabes an den Pedikelschrauben oftmals Verbindungshülsen bzw. sogenannte Down Tubes angebracht werden, welche eine provisorische Fixierung der polyaxialen Tulpen an den Pedikelschrauben oder ein Manipulieren der Wirbel ermöglichen, blockieren diese von den Pedikelschrauben abstehenden Verbindungshülsen die Schwenkbewegung des Stabeinsetzers beim senkrechten Einführen des Verbindungsstabes bzw. bei Schwenken des Stabeinsetzers. Zur Umgehung dieses Problems sieht der Stand der Technik sehr komplexe Klappmechanismen vor, mit denen man den Verbindungsstab relativ zum Stabeinsetzer verschwenken kann. Die relative Schwenkbarkeit des Verbindungsstabs zum Stabeinsetzer ermöglicht dann zwar das raumsparende Einsetzen eines Verbindungsstabes ohne gleichzeitig mit dem Stabeinsetzer an die Pedikelschrauben und / oder an Verbindungshülsen zur provisorischen Fixierung der Pedikelschrauben zu stoßen. Allerdings erfordern Klappmechanismen mehr Aufwand bei der Herstellung und mehr Aufwand bei der Sterilisation.

Zum anderen kann es bei der Benutzung bisheriger Stabeinsetzinstrumente problematisch sein, einen Verbindungsstab, der bereits in der Verbindungshülse einer ersten Pedikelschraube eingefädelt ist, zur Verbindungshülse einer benachbarten zweiten Pedikelschraube zu bewegen, um diesen auch dort einzufädeln.

Druckschrift US 2013/0041415 A1 zeigt ein Stabeinsetzinstrument, welches dafür vorgesehen ist, einen Verbindungsstab in Tulpen benachbarter Pedikelschrauben für eine dorsale Wirbelsäulenstabiliserung einzusetzen. Ein Gelenkmechanismus dreht dabei während eines Einsetzvorgangs den Verbindungsstab aus der Längsachse des Stabeinsetzinstruments heraus und positioniert ihn dadurch in einer vorgesehenen Lage in den Tulpen benachbarter Pedikelschrauben.

Die US 2005/0192589 A1 offenbart ein Stabeinsetzinstrument, bei dem sich zwei Hebel gabelförmig von einem langgestreckten Haltekopf, der über die Hälfte des Stabeinsetzinstruments ausmacht, weg erstrecken. Der Haltekopf mit angebundenem Verbindungsstab wird zur Positionierung durch bzw. in einen Durchgangsschlitz angebrachter Verbindungshülsen zur provisorischen Fixierung der Tulpen geführt. Bei diesem Stabeinsetzinstrument ist ein Verschrauben mittels einer Madenschraube in der Endstellung durch das Stabeinsetzinstrument verhindert.

### Offenbarung der Erfindung

Aufgabe der vorliegenden Erfindung ist es deshalb ein einfaches Stabeinsetzinstrument bereitzustellen, mit dessen Hilfe ein Verbindungsstab ohne unnötige Belastung des anliegenden Gewebes einsetzbar ist, und mit dem man das Einfädeln eines bereits in der Verbindungshülse einer ersten Pedikelschraube eingefädelten Verbindungsstabes in die Verbindungshülse und / oder Tulpe einer zweiten Pedikelschraube erleichtert.

Diese Aufgabe wird durch Merkmale des Anspruchs 1 gelöst. Vorteilhafte Weiterbildungen sind Gegenstand der Unteransprüche.

Ein erfindungsgemäßes Stabeinsetzinstrument weist einen Haltekopf, an dem ein Verbindungsstab, insbesondere mit Befestigungsmittel, lösbar befestigbar ist, und einen damit verbundenen Handhabungsabschnitt auf. Der Handhabungsabschnitt wird hierbei durch zwei Hebel bzw. Arme bzw. Hebelarme gebildet, die mit dem Haltekopf gabelförmig, insbesondere einstückig oder starr, verbunden.

Die Ausmaße des Haltekopfs in Längsrichtung des Stabeinsetzinstruments (d.h. in Proximal-Distal-Richtung) sind insbesondere kleiner als 40 Prozent der Gesamtlänge des Stabeinsetzinstruments (bzw. des Gesamtausmaßes des Stabeinsetzinstruments in Proximal-Distal-Richtung). Vorzugsweise ist dieser Anteil des Haltekopfs kleiner als 20 Prozent oder gar kleiner als 10 Prozent der Gesamtlänge des Stabeinsetzinstruments.

Bei dem Haltekopf handelt es sich also um einen kompakten Abschnitt am distalen Ende des Stabeinsetzinstruments, der sich nicht balkenförmig über einen verhältnismäßig großen Teil der Gesamtlänge des Stabeinsetzinstruments erstreckt. Insbesondere sind alle Ausmaße des Haltekopfs (also auch die in Proximal-Distal-Richtung des Stabeinsetzinstruments) kleiner als die Längserstreckung des einzusetzenden Verbindungsstabs (d.h. kleiner als der Abstand zwischen zwei zu verbindende Wirbeln).

Der Haltekopf ist insbesondere ringförmig und weist zumindest im Wesentlichen mittig eine Aufnahme für den Verbindungsstab auf. Die Hebel des Handhabungsabschnitts erstrecken sich insbesondere direkt von der Aufnahme im Haltekopf aus in Richtung zum proximalen Ende des Stabeinsetzinstruments.

Die zwei Hebel des Handhabungsabschnitts können insbesondere derart von einander beabstandet sein, dass die zwei Hebel beim Einsetzen des Verbindungsstabes die Pedikelschrauben und / oder daran angebrachte Verbindungshülsen zur provisorischen Fixierung der Pedikelschrauben zwischen sich aufnehmen können.

Somit lässt sich beim Einsetzen des Verbindungsstabes in Tulpen von benachbarten Pedikelschrauben das Stabeinsetzinstrument schwenken, ohne an die Pedikelschrauben und / oder an Verbindungshülsen zur provisorischen Fixierung der Pedikelschrauben anzustoßen beziehungsweise ohne von den Pedikelschrauben und / oder den Verbindungshülsen behindert zu werden. Diese Funktion wird dadurch gewährleistet, dass die zwei Hebel so ausgeformt und/oder so am Haltekopf angeordnet sind, dass sie die Pedikelschrauben und/oder die Verbindungshülsen beim Schwenken des Stabeinsetzinstruments während des Einsetzens des Verbindungsstabes zu beiden Seiten seitlich passieren bzw. umgreifen. Anders ausgedrückt sind die Hebel von einer Schwenkebene bzw. Befestigungsebene des Verbindungsstabs (Ebene, in welcher der an dem Stabeinsetzinstrument befestigte Verbindungsstab liegt) zu beiden Seiten beabstandet und zwar um mindestens den Durchmesser der Außenkontur der Pedikelschraube und / oder Verbindungshülse, so dass sie die Pedikelschraube bzw. Verbindungshülse zwischen sich aufnehmen können.

Erfindungsgemäß weist ein Stabeinsetzinstrument also eine gabelförmige Geometrie aus zwei Hebeln und einem Haltekopf auf, wobei die zwei Hebel insbesondere zu einer mittig durch den Haltekopf verlaufenden Ebene beabstandet und/oder symmetrisch ausgeformt sind.

Bei der vorliegenden Erfindung ist es von Vorteil, dass eine raumsparende Schwenkbarkeit des Stabeinsetzers während des Stabeinsetzvorgangs ohne einen Klappmechanismus zwischen den Hebeln und dem Haltekopf gewährleistet werden kann. Da im Rahmen des Stabeinsetzvorgangs zum Teil erhebliche Kräfte durch das Stabeinsetzinstrument übertragbar sein müssen, erweist sich die Einsparung eines den Anforderungen entsprechend sehr stabilen Klappmechanismus als besonders vorteilhaft. Die genannten Vorteile betreffen die einfachere Herstellbarkeit, die einfachere Sterilisation und die einfachere beziehungsweise die aufgrund des Fehlens eines Klappmechanismus nicht erforderliche Wartung eines erfindungsgemäßen Stabeinsetzers.

Des Weiteren ermöglicht die gabelförmige Ausbildung des erfindungsgemäßen Stabeinsetzinstruments die Ausrichtung des Stabeinsetzinstruments relativ zu zwei benachbarten Pedikelschrauben und umgekehrt. Die während eines Eingriffs an den Pedikelschrauben angebrachten Verbindungshülsen können zum einen als Führung für den erfindungsgemäßen Stabeinsetzer beim anfänglichen Einführen des Verbindungsstabes zur Tulpe der ersten Pedikelschraube dienen, und können zum anderen das Weiterführen des dann in der ersten Verbindungshülse eingefädelten Verbindungsstabes zur Tulpe der zweiten Pedikelschraube erleichtern. Die Führung des Stabeinsetzinstruments erfolgt dabei indem die Verbindungshülsen zwischen den zwei Hebeln eingeschlossen oder eingefasst werden oder seitlich kontaktiert werden.

Anderseits können benachbarte Verbindungshülsen über das Stabeinsetzinstrument ausgerichtet werden, wenn diese von beiden Seiten von den Hebelarmen des Stabeinsetzinstruments umgriffen werden. Dadurch kann sichergestellt werden, dass die Achsen der Verbindungshülsen, die mit den Achsen der Tulpen fluchten, die Achse der Aufnahme in dem Haltekopf und somit die Längsachse des einzusetzenden und in der Aufnahme befestigten Verbindungsstab in einer Ebene liegen. Durch die gegenseitige Führung bzw. Ausrichtung von Stabeinsetzinstrument und Verbindungshülsen bzw. Tulpen wird sichergestellt, dass der Verbindungsstab und die Aufnahmen in den Tulpen für den Verbindungsstab fluchten, so dass das Einführen des Verbindungsstabs in die Aufnahmen der Tulpen geführt und somit erleichtert wird.

Mittel, die ein Einfädeln des Verbindungsstabs in die Verbindungshülse beziehungsweise die Tulpe der zweiten Pedikelschraube erleichtern, sind bisher nur als zusätzliches Instrument bekannt. Durch die Verwendung eines erfindungsgemäßen Stabeinsetzers kann somit die Anzahl der notwendigen Instrumente ohne Funktionseinbußen verringert und die Durchführung eines Stabeinsetzvorgangs gleichzeitig vereinfacht werden.

Die grundsätzlichen Vorteile der vorliegenden Erfindung hinsichtlich Herstellung und Sterilisation können weiter verstärkt werden, wenn die Hebel beispielsweise durch Biegeumformen einstückig mit dem Haltekopf ausgebildet sind. Bei der Herstellung fallen dann die Arbeitschritte bezüglich der Anbindung der Hebel an den Haltekopf weg und können für die Sterilisierung des Stabeinsetzers ungünstige Fugen an den Verbindungsstellen vermieden werden.

Sind dem Haltekopf abgewandte Enden der Hebel eines Stabeinsetzinstruments miteinander verbunden, d.h. die Hebel haben ein geschlossenes Profil, kann die Stabilität des Stabeinsetzinstruments im Vergleich zu anderen Maßnahmen materialsparend erhöht werden. Alternativ oder zusätzlich können die beiden Hebel- bzw. Greifarme über ein oder mehrere Querstreben verbunden sein. Dies kann insbesondere hinsichtlich der durch den Stabeinsetzer zu übertragenden Kräfte sehr vorteilhaft sein. Dadurch wird sichergestellt, dass die Hebel- bzw. Greifarme beim Greifen oder Hantieren nicht so sehr zusammengedrückt werden, dass sie kraftschlüssig gegen die Verbindungshülsen gedrückt werden.

Alternativ oder zusätzlich können die dem Haltekopf abgewandten Enden der Hebel durch ein Griffteil verbunden werden. Wird das Griffteil lösbar, insbesondere werkzeuglos demontierbar, beispielsweise mithilfe von Steckverbindungen, an die Hebel angebunden, kann die Handhabbarkeit des Stabeinsetzinstruments nicht nur aufgrund des Vorhandenseins eines ausgewiesenen Griffstücks, sondern auch aufgrund von möglicher Variabilität der Anbindung des Griffteils an die Hebel verbessert werden. So kann das Griffteil beispielsweise so ausgeformt sein, dass es zum einen derart an die Hebel angebracht werden kann, dass es mit diesen im Wesentlichen fluchtet, und zum anderen derart, dass es senkrecht zu den Hebeln verläuft.

Um eine bessere Zugänglichkeit der Verbindungshülse von oben zu erreichen, z.B. zum Einführen eines Schraubendrehers oder einer anderen Vorrichtung in die Verbindungshülse, können die Hebel zumindest zwei zueinander angewinkelte Abschnitte aufweisen, so dass die proximalen Hebelenden, wenn der Verbindungsstab vollständig eingeführt ist, von den Verbindungshülsen wegragen, um so nicht das Fixieren des Verbindungsstabs in den Tulpen und ggf. das Entfernen der Verbindungshülsen zu behindern. Darüber hinaus kann die gekrümmte Ausformung der Hebel derart ausgeführt sein, dass das Verschwenken des Stabeinsetzers beim Stabeinsetzvorgang besonders ergonomisch erfolgen kann
Der Abstand von proximalen Hebelabschnitten zueinander kann größer als der Abstand von distalen Hebelabschnitten sein. Dies ist von Vorteil, da die an den Pedikelschrauben angebrachten Verbindungshülsen oftmals Abschnitte mit unterschiedlichen Durchmessern aufweisen und so sichergestellt wird, dass einerseits die distalen Hebelabschnitte an der Außenkontur der Verbindungshülsen seitlich anliegen oder zumindest ein geringes Spiel zu diesen haben und anderseits sich die proximalen Hebelabschnitte über die vergrößerten Köpfe der Verbindungshülsen führen bzw. verschwenken lassen.

Gemäß einem anderen oder zusätzlichen Aspekt kann der Haltekopf bezüglich der Hebel seitlich versetzt sein bzw. die Hebelabschnitte, die unmittelbar an den Haltekopf anschließen, können gekröpft sein. Dieser Versatz bzw. Offset des Haltekopfs zu den Hebelarmen entspricht vorzugsweise im Wesentlichen dem Abstand der Außenkontur der Verbindungshülse zur Längsachse der Verbindungshülse bzw. der Tulpe, so dass zumindest die distalen Abschnitt der Hebel, wenn der Verbindungsstab vollständig eingeführt ist, sich im Wesentlichen in Richtung der Verbindungshülsen erstrecken und daran seitlich anliegen. Dadurch wird erreicht, dass der Stabeinsetzer über die Hebel bis zum vollständigen Einsetzen des Verbindungsstabs zumindest durch eine Verbindungshülse seitlich geführt ist.

Ungeachtet der Art und Weise, wie der Verbindungsstab an ein erfindungsgemäßes Stabeinsetzinstrument befestigt wird, ob durch zumindest eine Feststellschraube, durch eine Klick- oder Klemmverbindung, kann durch die gekröpfte bzw. abgewinkelte Ausformung des Haltekopfs eine einfachere und bessere Zugänglichkeit für Instrumente zum Lösen der Befestigung des Verbindungsstabes an dem Stabeinsetzer von oben, in einer Richtung im Wesentlichen parallel zu den Hebeln, realisiert werden..

Um das anfängliche Führen des Verbindungsstabes zur Tulpe der ersten Pedikelschraube weiter zu vereinfachen beziehungsweise um die dazu notwendige Führung durch die umfassten Verbindungshälsen zu verbessern, ist es vorteilhaft, wenn Abschnitte des Haltekopfs so ausgeführt sind, dass sie als Führung beim Führen des Stabeinsetzinstruments entlang der Verbindungshülsen dienen können. Dies kann vor Allem durch einen Vorsprung am Haltekopf, der in den Raum zwischen den Hebeln hineinragt, insbesondere in der Mittelebene der beidem Hebel und der Aufnahme im Haltekopf liegt, bewerkstelligt werden. Während beim Führen des Verbindungsstabes zur Tulpe der ersten
Pedikelschraube entlang der Verbindungshülsen die Verbindungshülsen von den Hebeln des Stabeinsetzinstruments von außen her eingefasst werden, kann der Vorsprung am Haltekopf in seitliche Schlitze in den Verbindungshülsen eingreifen, um so eine genaue Relativpositionierung von dem Haltekopf bzw. des daran befestigten Verbindungsstabs und der Verbindungshülse zu erreichen.

Um die lösbare Anbindung des Verbindungsstabes an dem Stabeinsetzinstrument möglichst einfach und dennoch stabil umzusetzen, ist es von Vorteil, den Verbindungsstab mittels zumindest einer Befestigungsschraube am Haltekopf befestigbar zu machen. Vorzugsweise ist diese Befestigungsschraube so am Haltekopf angeordnet, dass sie in den Raum zwischen den Hebeln hineinragt und auch als Führung beim Führen des Stabeinsetzinstruments entlang der Verbindungshülsen zur Tulpe der ersten Pedikelschraube dienen kann. Mit anderen Worten kann der oben erwähnte Führungsvorsprung am Haltekopf durch die Befestigungsschraube realisiert werden.

Zusätzlich dazu ist es hinsichtlich der Einfachheit der Handhabung und der Schonung von umgebendem Gewebe vorteilhaft, wenn die Befestigungsschraube so am Haltekopf angeordnet ist, dass ein Schraubenzieher zum Öffnen und Schließen der Befestigungsschraube im Wesentlichen parallel zu den Hebeln an die Befestigungsschraube herangeführt werden kann. Somit kann zum Einführen des Schraubenziehers in das Operationsgebiet derselbe Zugang verwendet werden, der auch zum Einführen des Stabeinsetzinstruments verwendet wird.

Die vorliegende Erfindung betrifft auch ein Stabeinsetzset, das sich dafür eignet, im Rahmen einer dorsalen Wirbelsäulenstabilisierung einen Verbindungsstab in Tulpen benachbarter Pedikelschrauben einzusetzen. Das Stabeinsetzset weist ein Stabeinsetzinstrument gemäß einem der oben beschriebenen Aspekte und zumindest eine Verbindungshülse auf. Die Hebel des Handhabungsabschnitts des Stabeinsetzinstruments können die Verbindungshülse umgreifen und sind voneinander so beabstandet, dass die Hebel beim Verschwenken des Stabeinsetzinstruments während des Einsetzens des Verbindungsstabs an der Außenseite der Verbindungshülse geführt werden können.
Das erfindungsgemäße Stabeinsetzset kann auch eine zweite Verbindungshülse aufweisen, die entsprechend der ersten Verbindungshülse an der anderen Tulpe anbringbar ist, um diese Tulpe provisorisch zu fixieren. Die beiden Hebel des Handhabungsabschnitts des Stabeinsetzinstruments können die beiden Verbindungshülsen umgreifen und sind voneinander so beabstandet, dass die beiden Verbindungshülsen während des Einsetzens des Verbindungsstabs unmittelbar vor dem Verschwenken des Stabeinsetzinstruments mittels der Hebel relativ zueinander positioniert werden können, insbesondere fluchtend in der Schwenkebene des Stabeinsetzinstruments ausgerichtet werden können.

### Kurzbeschreibung der Zeichnungen

Die vorliegende Erfindung wird im Folgenden anhand bevorzugter Ausführungsbeispiele unter Bezugnahme auf die beigefügten Zeichnungen näher beschrieben. Es zeigen:
Figur 1a eine perspektivische Ansicht eines Stabeinsetzinstruments gemäß einer ersten Ausführungsform mit einem Verbindungsstab;
Figur 1b eine Explosionszeichnung eines Details des Stabeinsetzinstruments gemäß der ersten Ausführungsform mit einem Detail des Verbindungsstabes;
Figur 1c eine perspektivische Ansicht des Stabeinsetzinstruments gemäß der ersten Ausführungsform am Ende des Einsetzvorgangs mit den in zwei Pedikelschrauben eingesetzten Verbindungsstab und zwei an den Pedikelschrauben angebrachten Verbindungshülsen;
Figuren 2a bis 2h eine Sequenz perspektivischer Ansichten des Stabeinsetzinstruments gemäß einer zweiten Ausführungsform bei einem Einsetzvorgang des Verbindungsstabs in Pedikelschrauben unter Zuhilfenahme von Verbindungshülsen; und
Figuren 3a und 3b perspektivische Ansichten des Stabeinsetzinstruments gemäß einer dritten Ausführungsform.

### Detaillierte Beschreibung bevorzugter Ausführungsformen

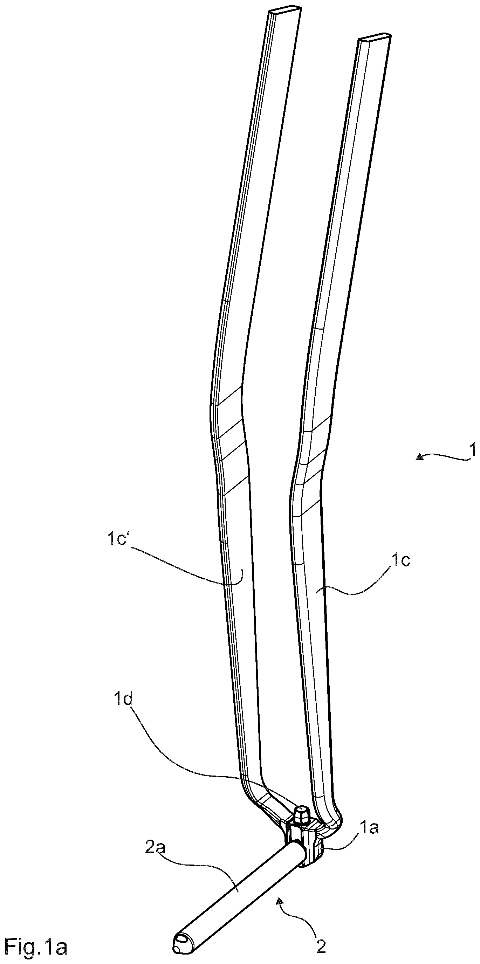

Figur 1a zeigt ein erfindungsgemäßes Stabeinsetzinstrument 1 gemäß einer ersten Ausführungsform der vorliegenden Erfindung.

Das Stabeinsetzinstrument 1 ist im Wesentlichen gabelförmig ausgebildet und weist an seinem distalen Ende einen Haltekopf 1a mit einer Ausnehmung 1b und zwei sich vom Haltekopf 1a aus gabelförmig wegerstreckenden Hebel 1c und 1c' auf.

Der Haltekopf 1a dient zur lösbaren Kopplung eines Verbindungsstabs 2, der in zumindest zwei benachbarte Pedikelschrauben 3 und 3' eingeführt und darin fixiert werden soll.

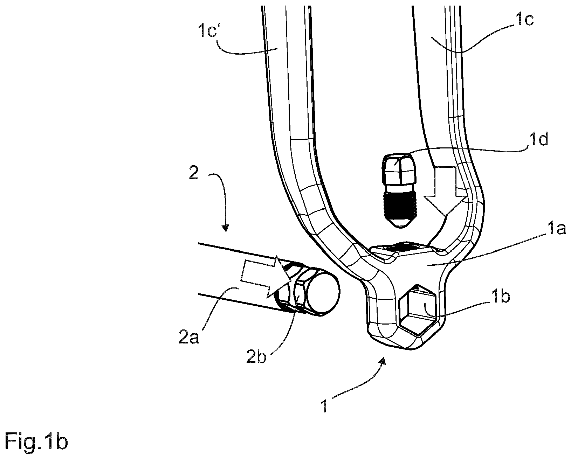

Wie in Figur 1b gezeigt, weist der Verbindungsstab 2, für den die im Folgenden beschriebenen Ausführungsformen des erfindungsgemäßen Stabeinsetinstruments 1 geeignet sein sollen, neben einem Schaftabschnitt 2a zur Verbindung von den zumindest zwei benachbarten Pedikelschrauben 3 und 3' an einem (proximalen) Ende einen Kopplungsabschnitt 2b, insbesondere mit einem polygonförmigen Querschnitt, z.B. Sechskantprofil, zum Koppeln des Verbindungsstabs 2 an das Stabeinsetzinstrument 1 auf.

Der Schaftabschnitt 2a gemäß der in den Figuren gezeigten Ausführungsform des Verbindungsstabs 2 ist kreiszylindrisch und gerade ausgeformt, kann aber auch gekrümmt ausgebildet sein.

Der Kopplungsabschnitt 2b kann in die Ausnehmung 1b, die an dem Haltekopf 1a des Stabeinsetzinstruments 1 vorgesehen ist, eingeführt werden. Insbesondere besitzt der Querschnitt der Ausnehmung 1b in Einführungsrichtung dieselbe Form wie der Kopplungsabschnitt 2b. In der vorliegenden Ausführungsform haben diese die Form eines Sechskantprofils. Die Ausmaße der Ausnehmung 1b sind vorzugsweise so gewählt, dass der Kopplungsabschnitt 2b des Verbindungsstabs 2 mit geringer Spielpassung in der Ausnehmung 1b aufgenommen beziehungsweise in die Ausnehmung 1b eingefügt werden kann. Aufgrund der nicht rotationssymmetrischen Querschnitte des Kopplungsabschnitts 2b und der Ausnehmung 1b ist der Verbindungsstab 2 drehfest zum Haltekopf 1a gesichert. Die Querschnitte des Kopplungsabschnitts 2b des Verbindungsstabs 2 und der Ausnehmung 1b des Haltekopfs 1a können einander so angepasst sein, dass sie nur eine bestimmte Drehorientierung zulassen, so dass der Verbindungsstab 2 nur in einer gewünschten Orientierung, was gerade bei gekrümmten Verbindungsstäben wichtig ist, in das Stabeinsetzinstrument 1 eingesetzt werden kann. Selbstverständlich können auch zwei oder mehrere Relativpositionen vorgesehen werden.

Die axiale Sicherung des Verbindungsstabs 2 im Haltekopf 1a erfolgt über eine Befestigungsschraube 1d, welche über eine radiale Gewindebohrung 1e, derart in die Ausnehmung 1b einschraubbar ist, dass diese den in die Ausnehmung 1b eingeführten Kopplungsabschnitt 2b kraftschlüssig fixiert. Die Gewindebohrung 1e mündet mittig zwischen den beiden Hebeln 1c und 1c', so dass der Kopf der Befestigungsschraube 1d mittig vom Haltekopf 1a aus zwischen die beiden Hebeln 1c und 1c' etwas vorragt. Der vorspringende bzw. vorragende Kopf der Befestigungsschraube 1d dient zur Führung des Stabeinsetzinstruments 1 beim Einsetzvorgang, was weiter unten detailliert beschrieben wird.

Die Arme bzw. Hebel 1c und 1c' sind einstückig mit dem Haltekopf 1a. Alternativ können die Hebel 1c und 1c' anderweitig an den Greifkörper 1a angebunden sein, beispielsweise durch eine Schraub-, Klebe- oder Schweißverbindung.

Die Hebel 1c und 1c' sind zueinander bezüglich einer durch die Mittelachse der Ausnehmung 1b verlaufenden Ebene symmetrisch.

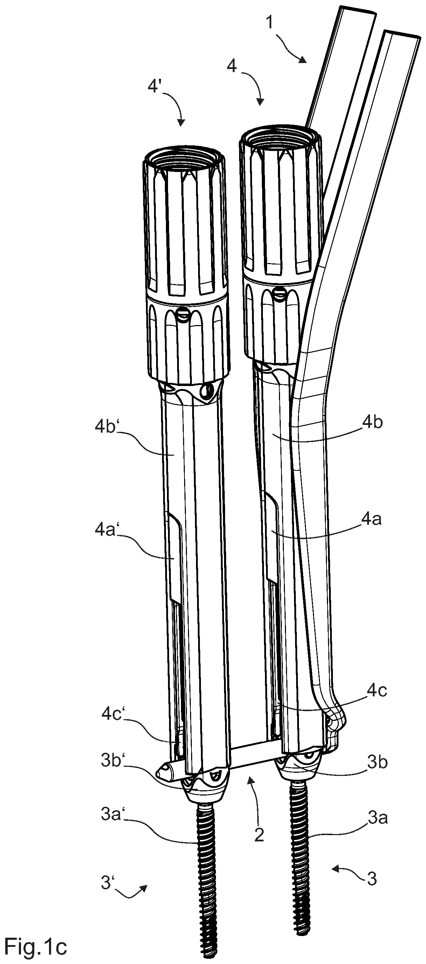

Die Hebel 1c und 1c' verlaufen im Wesentlichen parallel zueinander, wobei offene bzw. proximale Endabschnitte der Hebel 1c und 1c' bezüglich medialer Abschnitte der Hebel 1c und 1c' leicht abgewinkelt sind und distale Endabschnitte der Hebel 1c und 1c' bzw. die Enden, die mit dem Haltekopf 1a verbunden sind, derart gekröpft ausgebildet sind, dass der Haltekopf 1a zu den medialen Abschnitten der Hebel 1c und 1c' seitlich versetzt bzw. parallel verschoben ist. Dieser Abstand ist so gewählt, dass, wenn das Stabeinsetzinstrument 1, wie in der Figur 1c gezeigt, in einer Position ist, in der der Verbindungsstab 2 in die Pedikelschrauben 3, und 3' vollständig eingesetzt ist, der Haltekopf 1a seitlich an Verbindungshülsen 4 und 4', die zur provisorischen Fixierung der Tulpe 3b und 3b' angebracht sind, anliegt, und die medialen Abschnitten der Hebel 1c und 1c' ebenfalls seitlich (von anderen Seiten) an den Verbindungshülsen 4 und 4' anliegen.

Der Abstand zwischen den proximalen Endabschnitten ist größer als zwischen den medialen Abschnitten, wobei der Abstand in den jeweiligen Abschnitten im Wesentlichen konstant ist. Ausgehend vom Haltekopf 1a vergrößert sich also der Abstand zwischen den Hebeln 1c und 1c' zunächst mit zunehmender Entfernung vom Haltekopf 1a, bleibt dann im Bereich der medialen Abschnitte der Hebel 1c und 1c' gleich, vergrößert sich dann wieder etwas, und bleibt dann im Bereich der proximalen Abschnitte der Hebel 1c und 1c' wieder konstant.

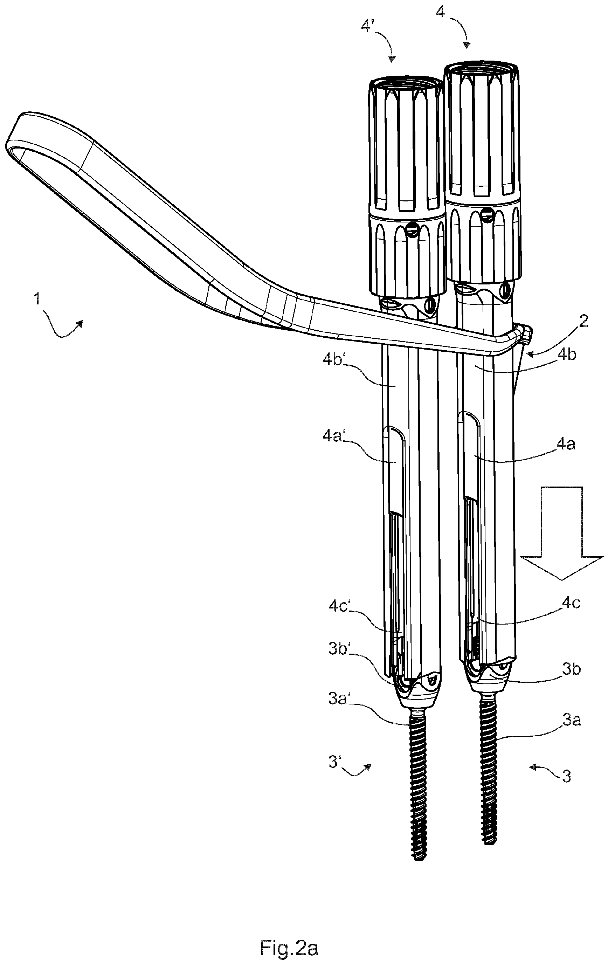

Figuren 2a bis 2h zeigen eine zweite Ausführungsform des Stabeinsetzinstruments 1, die sich von der ersten Ausführungsform lediglich dadurch unterscheidet, dass die proximalen bzw. vom Haltekopf 1a entfernten Enden der Hebel 1c und 1c' miteinander verbunden sind, genauer gesagt über ein abgerundetes bzw. halbringförmiges Verbindungsstück.

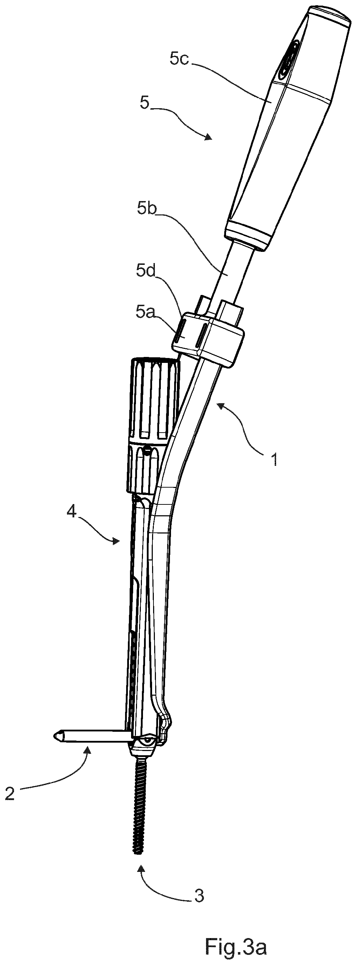

Gemäß einer in den Figuren 3a bis 3c gezeigten dritten Ausführungsform werden die dem Haltekopf 1a abgewandten offenen bzw. proximalen Enden der Hebel 1c und 1c' miteinander mittels eines separaten Griffteils 5 verbunden. Folglich unterscheidet sich die dritte Ausführungsform von der ersten Ausführungsform nur dahingehend, dass sie eine weitere Komponente in Form des Griffteils 5 aufweist.

Das Griffteil 5 weist ein im Wesentlichen quaderförmiges Verbindungsstück 5a auf, das über einen Stiel 5b mit einem ergonomisch geformten Griff 5c verbunden ist. Das Verbindungsstück 5a, der Stiel 5b und/oder der Griff 5c können einstückig oder mehrstückig ausgebildet sein.

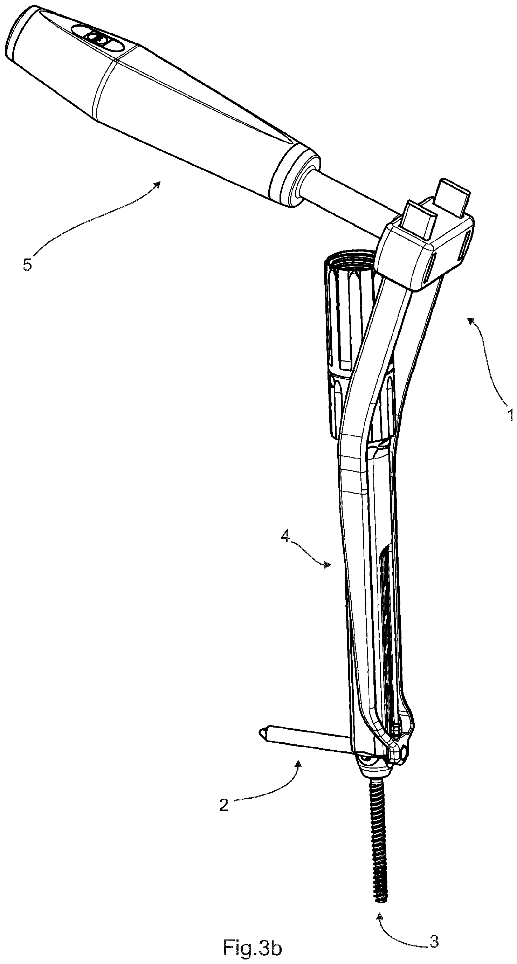

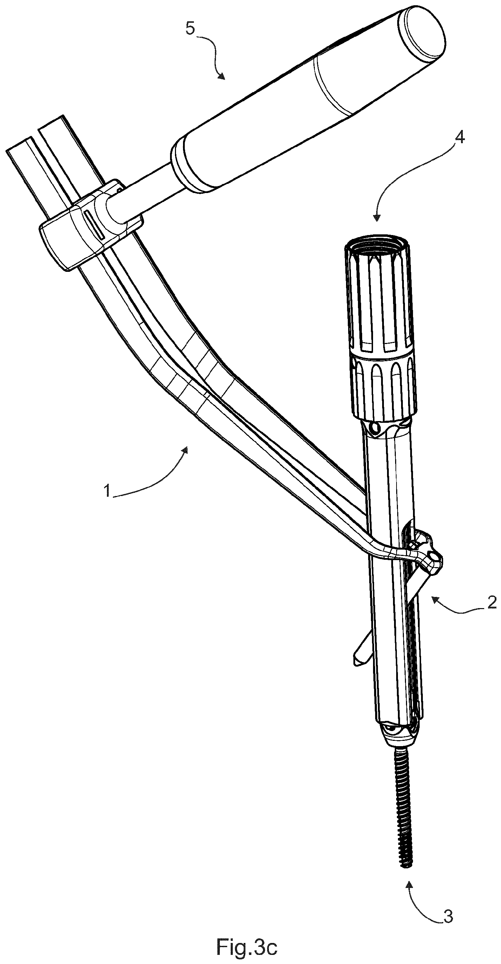

Um das Griffteil 5 an die beiden Hebel 1c und 1c' anzubinden, sind an dem Verbindungsstück 5a mehrere auf unterschiedlichen Seiten angeordnete Paare von Aufnahmen bzw. Schlitze 5d vorgesehen, in die jeweils die offenen bzw. proximalen Enden der Hebel 1c und 1c' gesteckt werden können. Wie ein Vergleich der Figuren 3a, 3b und 3c zeigt, kann aufgrund der Vielzahl an Aufnahmen 5d das Griffteil 5 in unterschiedlichen Orientierungen relativ zu den Hebeln 1c und 1c' mit diesen verbunden werden. Das Verbindungsstück 5a kann lediglich, insbesondere reibschlüssig, aufgesteckt sein. Die Aufnahmen 5d bzw. Hebel 1c und 1c' können auch dergestalt sein, z.B. leicht konisch oder mit Anschlag, dass das Verbindungsstück 5a nur eine bestimmte Strecke aufgeschoben werden kann. Ferner können (nicht gezeigte) Klemmverbindungen zur Fixierung von Verbindungsstück 5a und Hebel 1c und 1c' vorgesehen sein.

Die in den Figuren 1a bis 3b gezeigten und oben beschriebenen Ausführungsformen des erfindungsgemäßen Stabeinsetzinstruments 1 stellen lediglich drei mögliche Umsetzungen der beanspruchten Erfindung dar.

Im Folgenden wird die Handhabung des Stabeinsetzinstruments 1, insbesondere zum Einsetzen eines Verbindungsstabes 2 in Tulpen 3b, 3b' benachbarter Pedikelschrauben 3 und 3' anhand der Figuren 2a bis 2g beschrieben.

Die Figuren 2a bis 2h zeigen beispielhaft einen Stabeinsetzvorgang mithilfe eines Stabeinsetzinstruments 1 gemäß der zweiten Ausführungsform, wobei Analoges auch für die erste und dritte Ausführungsform gilt.

Ausgangspunkt sind eine erste und eine zweite, zueinander benachbarte polyaxiale Pedikelschrauben 3 und 3', deren Schäfte 3a und 3a' in Pedikel zweier benachbarter Wirbel (nicht gezeigt) eingedreht sind und deren Tulpen 3b und 3b' mittels Verbindungshülsen 4 und 4' provisorisch fixiert sind. Die Fixierung der Tulpe 3b oder 3b' bezüglich des entsprechenden Schafts 3a oder 3a' wird dadurch erreicht, dass ein Innenteil 4a oder 4a' der Verbindungshülse 4 oder 4' gegen das entsprechende Außenteil 4b oder 4b' verspannt wird, was wiederum eine Verspannung der Tulpe 3b oder 3b' relativ zum Schraubenkopf bzw. Schaft 3a oder 3a' zur Folge hat. Die Fixierung der Tulpen 3b und 3b' erfolgt so, dass Seitenschlitze 4c und 4c' der Verbindungshülsen 4 und 4' miteinander fluchten. Die Seitenschlitze 4c und 4c' dienen zum Durchführen bzw. Durchschwenken des Verbindungsstabs 2.

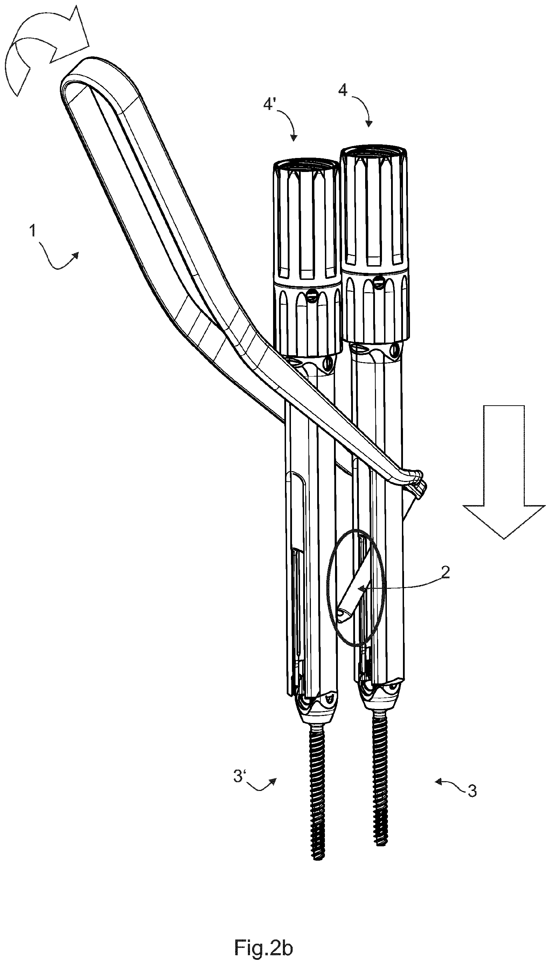

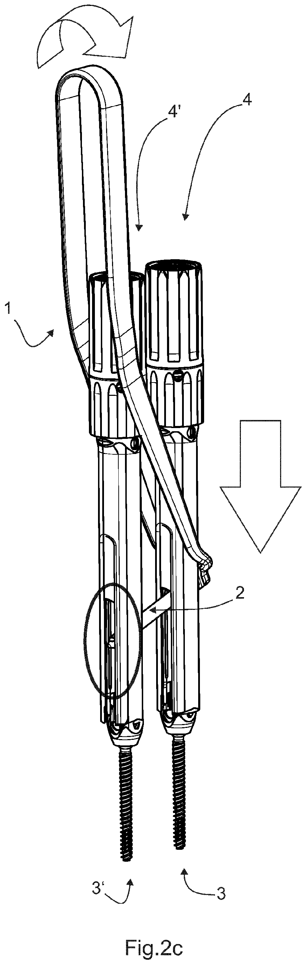

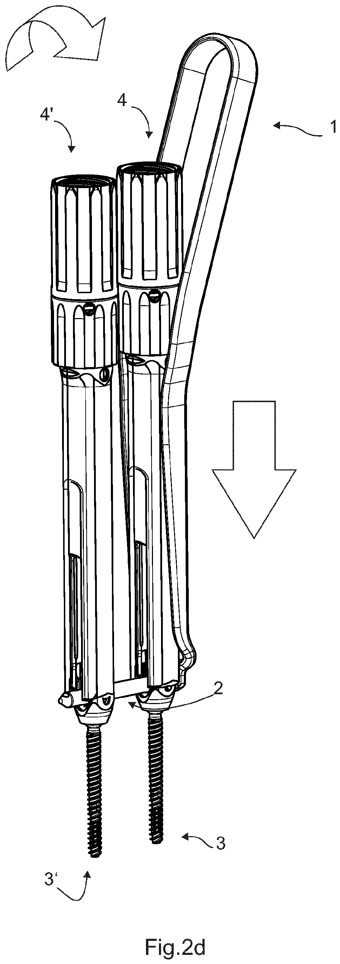

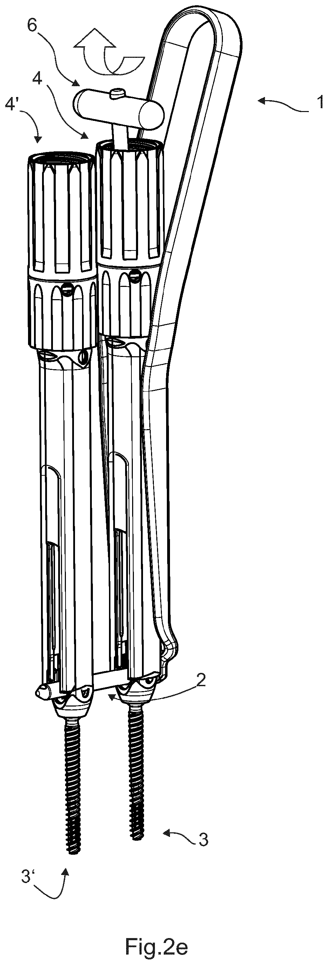

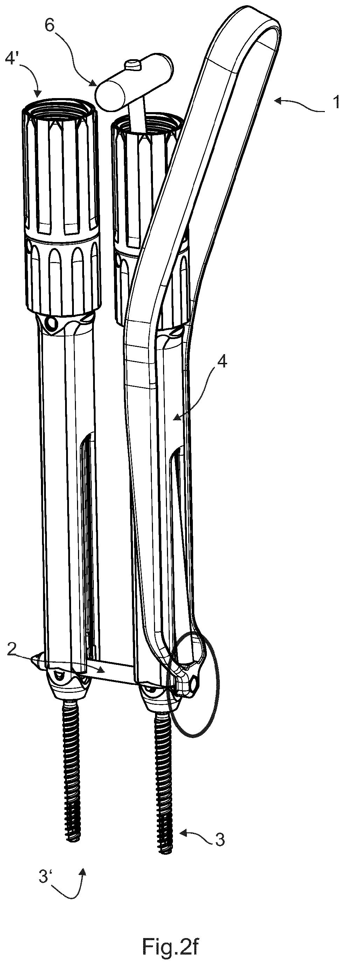

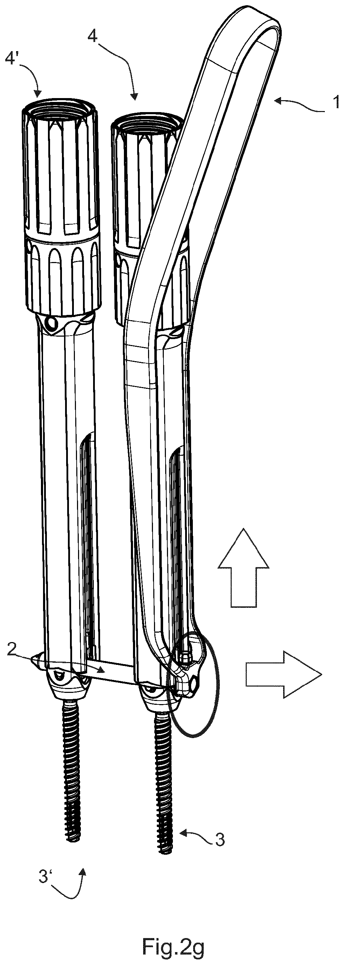

Das Stabeinsetzinstrument 1 wird über den proximalen, abgewinkelte (Greif-) Abschnitt, an dem die Hebel 1c und 1c' weiter von einander beabstandet sind, über die verbreiterten Köpfe der Verbindungshülsen 4 und 4' derart geführt, dass der Verbindungsstab 2, der mittels der Befestigungsschraube 1d an dem Haltekopf 1a lösbar befestigt ist, nach unten bzw. zu den Pedikel weist. Anschließend wird das eingefädelte Stabeinsetzinstrument 1 im Wesentlichen quer (nach links in der Figur 2) zu den Verbindungshülsen 4 und 4' verschoben, so dass der Haltekopf 1a, genauer gesagt der nach innen vorspringende Kopf der Befestigungsschraube 1d, in Kontakt mit der einen (in Figur 2a rechten) Verbindungshülse 4 kommt und die weniger von einander beabstandeten medialen Abschnitte der Hebel 1c und 1c' Außenteile 4b oder 4b' von gegenüberliegenden Seiten mit geringem Spiel umfassen und kontaktieren. Die Figur 2a zeigt den Zustand nach dem Einfädeln und der Querverschiebung des Stabeinsetzinstruments 1.

Anschließend wird Stabeinsetzinstrument 1 an den Außenteilen 4b oder 4b' nach unten bzw. in Richtung der Tulpe 3b der ersten Pedikelschraube 3 geführt. Gleichzeitig kann die relativen Lage der Verbindungshülsen 4 und 4' zueinander mithilfe des Stabeinsetzinstruments 1 ausgerichtet werden beziehungsweise kann die Ausrichtung der fixierten Verbindungshülsen 4 und 4' korrigiert werden. Da die Hebel 1c und 1c' des Stabeinsetzinstruments 1 die Verbindungshülsen 4 und 4' umgreifen, kann zum einen der Verbindungsstab 2 in der Nähe und im Wesentlichen parallel zu der Verbindungshülse 4 der ersten Pedikelschraube 3 geführt werden und können sich zum anderen die Hebel 1c und 1c' ebenfalls in der Nähe aber im Wesentlichen senkrecht zu den Verbindungshülsen 4 und 4' erstrecken (siehe Figur 2a).

Die Größe des vom Haltekopf 1a nach innen bzw. zwischen die beiden Hebel 1c und 1c etwas vorragenden Kopfs der Befestigungsschraube 1d ist an die Weite der Seitenschlitze 4c und 4c', die in den Verbindungshülsen 4 und 4' ausgebildet sind und in Längsrichtung verlaufen, angepasst.

Sobald der Kopf der Befestigungsschraube 1d am oberen Ende des entsprechenden Seitenschlitzes 4 der ersten Pedikelschraube 3 eingreift, ist das Stabeinsetzinstrument 1 vertikal bzw. in Längserstreckung der Verbindungshülsen 4 und 4' geführt und gegen Verdrehen gesichert, so dass beim anschließenden Verschwenken des Stabeinsetzinstruments 1 und des daran befestigten Verbindungsstabs 2 durch die Seitenschlitze 4c der ersten Verbindungshülse 4 in Richtung der Verbindungshülse 4' der zweiten Pedikelschraube 3', die Längsachse des Verbindungsstabs 2, die Seitenschlitze 4c und 4c' der beiden Verbindungshülsen 4 und 4' und somit die Tulpen 3b und 3b' der beiden Pedikelschrauben 3 in der Schwenkebene des Stabeinsetzinstruments 1 liegen.

Die vor dem Verschwenken im Wesentlich parallel zur und nahe der Verbindungshülse 4 nach unten geführte Verbindungsstab 2 kann unmittelbar neben der Verbindungshülse 4 über dieselbe für das Einsetzen der Pedikelschraube 3 vorgenommene Inzision in den Körper eingeführt werden, so dass keine weitere Inzision notwendig ist. (siehe Figur 2b).

Anschließend kann der Verbindungsstab 2 in die Seitenschlitze 4' der Verbindungshülse 4' der zweiten Pedikelschraube 3' schwenkend eingeführt werden (siehe Figur 2c) und in die beide Tulpen 3b und 3b' der beiden Pedikelschrauben 3 und 3' gedrückt werden (siehe Figur 2d).

Mittels eines Schraubenziehers 6 kann dann über das hohle Innere der Verbindungshülse 4' eine Feststellschraube bzw. set screw 3c' (nicht gezeigt) in die Tulpe 3b' gedreht werden, wodurch der Verbindungsstab 2 gegenüber der zweiten Pedikelschraube 3' fixiert wird. Genauso kann dann mithilfe des Schraubenziehers 6 über das hohle Innere der Verbindungshülse 4 eine Feststellschraube 3c (nicht gezeigt) in die Tulpe 3b gedreht werden, wodurch der Verbindungsstab 2 auch gegenüber der ersten Pedikelschraube 3' fixiert ist (siehe Figur 2e). Da der proximale Abschnitt des Stabeinsetzinstruments 1 bzw. die proximalen Enden der Hebel 1c und 1c' abgewinkelt sind, wird der Zugang und die Handhabung des Schraubenziehers 6 erleichtert.

Bevor der Schraubenzieher 6 wieder vollständig aus der Verbindungshülse 4 gezogen wird, kann dieser nach dem Festziehen der Feststellschraube 3c etwas angehoben und so geschwenkt werden, dass er durch den dem Haltekopf 1a des Stabeinsetzinstruments 1 zugewandten Seitenschlitz 4 der Verbindungshülse 4 greift, um mithilfe des Schraubenziehers 6 die Befestigungsschraube 1d vom Haltekopf 1a zu lösen und den Verbindungsstab 2 freizugeben (siehe Figur 2f).

Anschließend kann der Stabeinsetzer 1 leicht zurückgeschwenkt oder seitlich von der Pedikelschraube 3 weggeführt werden (in der Figur 2g nach rechts), so dass der Kopplungsabschnitt 2b des Verbindungsstabs 2 aus der Ausnehmung 1b des Haltekopfs 1a gezogen wird, um abschließend das Stabeinsetzinstrument 1 aus dem Operationsgebiet zu entfernen (siehe Figur 2g). Schließlich können Verbindungshülsen 4 und 4' von den Pedikelschrauben 3 und 3' gelöst werden.

Die Erfindung wurde zuvor anhand bevorzugter Ausführungsformen beschrieben, ist jedoch hierauf nicht beschränkt. Gemäß dem zweiten Ausführungsbeispiel lässt sich über denselben Schraubenzieher 6 sowohl die Feststellschrauben bzw. set screws 3c und 3c' der Pedikelschraube 3 und 3' festziehen als auch die Befestigungsschraube 1d des Haltekopfs 1a lösen. Selbstverständlich können sich die Schrauben unterscheiden und diese mit unterschiedlichen Schraubenziehern befestigt bzw. gelöst werden.

Ferner können die Hebel 1c und 1c' so lange sein, dass sie mehr als zwei Verbindungshülsen 4 benachbarter Pedikelschrauben 3 umgreifen.

**Bezugszeichenliste**

| | |
|---|---|
| Stabeinsetzinstrument | 1 |
| Haltekopf | 1a |
| Ausnehmung | 1b |
| Hebel | 1c, 1c' |
| Befestigungsschraube | 1d |
| Gewindebohrung | 1e |
| Verbindungsstab | 2 |
| Schaftabschnitt | 2a |
| Kopplungsabschnitt | 2b |
| Pedikelschraube | 3, 3' |
| Schaft | 3a, 3a' |
| Tulpe | 3b, 3b' |
| Feststellschraube | 3c, 3c' |
| Verbindungshülse | 4, 4' |
| Innenteil | 4a, 4a' |
| Außenteil | 4b, 4b' |
| Seitenschlitz | 4c, 4c' |
| Griffteil | 5 |
| Verbindungsstück | 5a |
| Stiel | 5b |
| Griff | 5c |
| Aufnahme | 5d |
| Schraubenzieher | 6 |

## Patentansprüche

1. Stabeinsetzinstrument (1) zum Einsetzen eines Verbindungsstabes (2) in Tulpen (3b, 3b') benachbarter Pedikelschrauben (3, 3') im Rahmen einer dorsalen Wirbelsäulenstabilisierung, mit einem am distalen Ende des Stabeinsetzinstruments (1) angeordneten Haltekopf (1a), an dem der Verbindungsstab (2), insbesondere mit Befestigungsmittel (1d), lösbar befestigbar ist, und einem damit verbundenen Handhabungsabschnitt, welcher durch zwei mit dem Haltekopf (1a), insbesondere einstückig oder starr, verbundene Hebel (1c, 1c') gebildet wird,
**dadurch gekennzeichnet, dass**
sich die Hebel (1c, 1c') gabelförmig vom Haltekopf (1a) weg erstrecken und derart voneinander beabstandet sind, dass diese beim Einsetzen des Verbindungsstabes (2) in die Tulpen (3b, 3b') die Pedikelschrauben (3, 3') und daran angebrachte Verbindungshülsen (4, 4') zur provisorischen Fixierung der Tulpen (3b, 3b') zwischen sich aufnehmen können.

2. Stabeinsetzinstrument (1) gemäß Anspruch 1, **dadurch gekennzeichnet, dass** von dem Haltekopf (1a) abgewandte Enden der Hebel (1c, 1c') miteinander verbunden sind.

3. Stabeinsetzinstrument (1) gemäß Anspruch 1, **dadurch gekennzeichnet, dass** von dem Haltekopf (1a) abgewandte offene Enden der Hebel (1c, 1c') durch ein, insbesondere lösbar an den offenen Enden der Hebel (1c, 1c') anbringbares, Griffteil (5) verbunden sind.

4. Stabeinsetzinstrument (1) gemäß einem der Ansprüche 1 bis 3, **dadurch gekennzeichnet, dass** die Hebel (1c, 1c') abschnittsweise gekröpft und/oder abgewinkelt ausgebildet sind.

5. Stabeinsetzinstrument (1) gemäß einem der Ansprüche 1 bis 4, **dadurch gekennzeichnet, dass** die Hebel (1c, 1c') einen ersten Abschnitt aufweisen, in dem sich diese parallel zueinander erstrecken und einen ersten Abstand zueinander haben, und einen zweiten Abschnitt aufweisen, in dem der Abstand der Hebel (1c, 1c') zueinander größer als der erste Abstand ist, wobei der zweite Abschnitt auf der vom Haltekopf (1a) abgewandeten Seite des ersten Abschnitts angeordnet ist.

6. Stabeinsetzinstrument (1) gemäß einem der Ansprüche 1 bis 5, **dadurch gekennzeichnet, dass** der Haltekopf (1a) eine Aufnahme (1b) zur lösbaren Befestigung des Verbindungsstabes (2) aufweist, wobei die Achse der Aufnahme (1b) senkrecht zur Längserstreckung der Hebel (1c, 1c'), insbesondere zur Längserstreckung der Hebel (1c, 1c') in dem ersten Abschnitt, verläuft.

7. Stabeinsetzinstrument (1) gemäß einem der Ansprüche 1 bis 6, **dadurch gekennzeichnet, dass** zumindest ein Abschnitt am Haltekopf (1a) derart ausgebildet ist, dass er als Führung beim Führen des Stabeinsetzinstruments (1) entlang der Verbindungshülsen (4, 4') dienen kann, insbesondere zwischen den Hebeln (1c, 1c') ein Führungszapfen ausgebildet ist, der in einen entsprechenden Seitenschlitz (4c) der Verbindungshülse (4) eingreifen kann.

8. Stabeinsetzinstrument (1) gemäß Anspruch 7, **dadurch gekennzeichnet, dass** der Führungszapfen durch einen Schraubenkopf einer Befestigungsschraube (1d) gebildet wird, mittels welcher der Verbindungsstab (2) am Haltekopf (1a) befestigbar ist.

9. Stabeinsetzinstrument (1) gemäß einem der Ansprüche 1 bis 8, **dadurch gekennzeichnet, dass** der Haltekopf (1a) ringförmig ausgebildet ist.

10. Stabeinsetzset (1, 4), mit:
einem Stabeinsetzinstrument (1) gemäß einem der Ansprüche 1 bis 9, und
einer Verbindungshülse (4), die an einer der Tulpen (3b, 3b') anbringbar ist, um die Tulpe (3b) provisorisch zu fixieren,
**dadurch gekennzeichnet, dass**
die beiden Hebel (1c, 1c') des Handhabungsabschnitts des Stabeinsetzinstruments (1) die Verbindungshülse (4) umgreifen können und voneinander so beabstandet sind, dass die Hebel (1c, 1c') beim Verschwenken des Stabeinsetzinstruments (1) während des Einsetzens des Verbindungsstabs (2) an der Außenseite der Verbindungshülse (4) geführt werden können.

11. Stabeinsetzset (1, 4, 4') gemäß Anspruch 10, **dadurch gekennzeichnet, dass** eine zweite Verbindungshülse (4') vorgesehen ist, die entsprechend an der anderen Tulpe (3b') anbringbar ist, um diese Tulpe (3b') provisorisch zu fixieren, und dass die beiden Hebel (1c, 1c') des Handhabungsabschnitts des Stabeinsetzinstruments (1) die beiden Verbindungshülsen (4, 4') umgreifen können und voneinander so beabstandet sind, dass die beiden Verbindungshülsen (4, 4') während des Einsetzens des Verbindungsstabs (2) unmittelbar vor dem Verschwenken des Stabeinsetzinstruments (1) mittels der Hebel (1c, 1c') relativ zueinander positioniert werden können.

## Claims

1. Rod insertion instrument (1) for inserting a connecting rod (2) into tulips (3b, 3b') of adjacent pedicle screws (3, 3') as part of a dorsal spine stabilisation, with a holding head (1a) arranged at the distal end of the rod insertion instrument (1) to which the connecting rod (2) can be detachably fastened, in particular with fastening means (1d), and a handling section connected thereto, which is formed by two levers (1c, 1c') connected to the holding head (1a), in particular integrally or rigidly,
**characterised in that**
the levers (1c, 1c') extend away fork-shaped from the holding head (1a) and are spaced apart from one another in such a way that when the connecting rod (2) is inserted into the tulips (3b, 3b'), the pedicle screws (3, 3') and the connection sleeves (4, 4') mounted thereon for provisional fixation of the tulips (3b, 3b') can be received between them.

2. Rod insertion instrument (1) according to claim 1, **characterised in that** the ends of the levers (1c, 1c') facing away from the holding head (1a) are connected to one another.

3. Rod insertion instrument (1) according to claim 1, **characterised in that** the open ends of the levers (1c, 1c') facing away from the holding head (1a) are connected by a grip part (5) which can be mounted, in particular releasably, at the open ends of the levers (1c, 1c').

4. Rod insertion instrument (1) according to one of claims 1 to 3, **characterised in that** the levers (1c, 1c') are designed offset and/or angled in sections.

5. Rod insertion instrument (1) according to one of claims 1 to 4, **characterised in that** the levers (1c, 1c') have a first section in which these extend parallel to one another and have a first distance to one another, and have a second section in which the distance of the levers (1c, 1c') to one another is greater than the first distance, wherein the second section is arranged on the side of the first section facing away from the holding head (1a).

6. Rod insertion instrument (1) according to one of claims 1 to 5, **characterised in that** the holding head (1a) has a recess (1b) for releasable attachment of the connecting rod (2), wherein the axis of the recess (1b) extends perpendicular to the longitudinal extension of the levers (1c, 1c'), in particular to the longitudinal extension of the levers (1c, 1c') in the first section.

7. Rod insertion instrument (1) according to one of claims 1 to 6, **characterised in that** at least one section is formed on the holding head (1a) in such a way that it can act as a guide by guiding the rod insertion instrument (1) along the connection sleeve (4, 4'), in particular a guide pin is formed between the levers (1c, 1c'), which can engage in a corresponding side slot (4c) of the connection sleeve (4).

8. Rod insertion instrument (1) according to claim 7, **characterised in that** the guide pin is formed by a screw head of a fastening screw (1d), by means of which the connecting rod (2) can be fastened to the holding head (1a).

9. Rod insertion instrument (1) according to one of claims 1 to 8, **characterised in that** the holding head (1a) is formed ring-shaped.

10. Rod insertion set (1, 4), comprising:
a rod insertion instrument (1) according to one of claims 1 to 9, and a connection sleeve (4), which can be mounted on one of the tulips (3b, 3b') in order to provisionally fix the tulip (3b),
**characterised in that**
both levers (1c, 1c') of the handling section of the rod insertion instrument (1) can surround the connection sleeve (4) and are spaced apart from each other in such a way that when the rod insertion instrument (1) is pivoted during the insertion of the connecting rod (2), the levers (1c, 1c') can be guided on the outside of the connection sleeve (4).

11. Rod insertion set (1, 4, 4') according to claim 10, **characterised in that** a second connection sleeve (4') is provided which can be correspondingly mounted on the other tulip (3b'), in order to provisionally fix this tulip (3b'), and **in that** the two levers (1c, 1c') of the handling section of the rod insertion instrument (1) can surround both connection sleeves (4, 4') and are spaced apart from each other, in such a way that the two connection sleeves (4, 4') can be positioned relative to one another during the insertion of the connecting rod (2) directly before the pivoting of the rod insertion instrument (1) by means of the levers (1c, 1c').

## Revendications

1. Instrument d'insertion de tiges (1) destiné à insérer une tige de liaison (2) dans des vis pédiculaires (3, 3') adjacentes à des tulipes (3b, 3b') dans le cadre d'une stabilisation de la colonne vertébrale dorsale, muni d'une tête de retenue (1a) disposée à l'extrémité distale de l'instrument d'insertion de tiges (1), à laquelle la tige de liaison (2) peut être fixée de manière amovible, en particulier avec un moyen de fixation (1d), et d'une section de préhension reliée à celui-ci, laquelle est formée par deux leviers (1c, 1c'), en particulier d'une seule pièce ou rigide, reliés à la tête de retenue (1a),
**caractérisé en ce que**
les leviers (1c, 1c') s'éloignent en forme de fourche de la tête de retenue (1a) et sont espacés l'un de l'autre de sorte que ceux-ci peuvent recevoir entre eux lors de l'insertion de la tige de liaison (2) dans les tulipes (3b, 3b') les vis pédiculaires (3, 3') et des douilles de liaison (4, 4') montées sur ceux-ci pour fixer provisoirement les tulipes (3b, 3b').

2. Instrument d'insertion de tiges (1) selon la revendication 1, **caractérisé en ce que** des extrémités des leviers (1c, 1c') opposées à la tête de retenue (1a) sont reliées entre elles.

3. Instrument d'insertion de tiges (1) selon la revendication 1, **caractérisé en ce que** des extrémités ouvertes des leviers (1c, 1c') opposées à la tête de retenue (1a) sont reliées par une partie de préhension (5) pouvant être montée, en particulier de manière amovible aux extrémités ouvertes des leviers (1c, 1c').

4. Instrument d'insertion de tiges (1) selon l'une quelconque des revendications 1 à 3, **caractérisé en ce que** les leviers (1c, 1c') sont formés par tronçons de manière coudée et/ou recourbée.

5. Instrument d'insertion de tiges (1) selon l'une quelconque des revendications 1 à 4, **caractérisé en ce que** les leviers (1c, 1c') présentent un premier tronçon, dans lequel ceux-ci s'étendent parallèlement l'un par rapport à l'autre et présentent une première distance entre eux et présentent un deuxième tronçon, dans lequel la distance entre les leviers (1c, 1c') est supérieure à la première distance, le deuxième tronçon étant disposé sur le côté opposé à la tête de retenue (1a) du premier tronçon.

6. Instrument d'insertion de tiges (1) selon l'une quelconque des revendications 1 à 5, **caractérisé en ce que** la tête de retenue (1a) présente un logement (1b) pour fixer de manière amovible la tige de liaison (2), l'axe du logement (1b) s'étendant perpendiculairement à l'étendue longitudinale des leviers (1c, 1c'), en particulier à l'étendue longitudinale des leviers (1c, 1c') dans le premier tronçon.

7. Instrument d'insertion de tiges (1) selon l'une quelconque des revendications 1 à 6, **caractérisé en ce qu'**au moins un tronçon de la tête de retenue (1a) est formé de sorte qu'il peut servir de guide lors du guidage de l'instrument d'insertion de tiges (1) le long des douilles de liaison (4, 4'), en particulier un tourillon de guidage est formé entre les leviers (1c, 1c'), qui peut entrer en prise avec une fente latérale (4c) correspondante de la douille de liaison (4).

8. Instrument d'insertion de tiges (1) selon la revendication 7, **caractérisé en ce que** le tourillon de guidage est constitué par une tête de vis d'une vis de fixation (1d), au moyen de laquelle la tige de liaison (2) peut être fixée à la tête de retenue (1a).

9. Instrument d'insertion de tiges (1) selon l'une quelconque des revendications 1 à 8, **caractérisé en ce que** la tête de retenue (1a) est réalisée sous la forme d'un anneau.

10. Ensemble d'insertion de tiges (1, 4), comprenant :
un instrument d'insertion de tiges (1) selon l'une quelconque des revendications 1 à 9, et
une douille de liaison (4) qui peut être montée sur l'une des tulipes (3b, 3b'), pour fixer provisoirement la tulipe (3b),
**caractérisé en ce que**
les deux leviers (1c, 1c') de la section de préhension de l'instrument d'insertion de tiges (1) peuvent entourer la douille de liaison (4) et sont espacés l'un de l'autre de telle sorte que les leviers (1c, 1c') peuvent être guidés lors du pivotement de l'instrument d'insertion de tiges (1) pendant l'insertion de la tige de liaison (2) au niveau du côté externe de la douille de liaison (4).

11. Ensemble d'insertion de tiges (1, 4, 4') selon la revendication 10, **caractérisé en ce qu'**une seconde douille de liaison (4') est prévue, qui peut être montée de manière correspondante à l'autre tulipe (3b'), pour fixer provisoirement cette tulipe (3b'), et **en ce que** les deux leviers (1c, 1c') de la section de préhension de l'instrument d'insertion de tiges (1) peuvent entourer les deux douilles de liaison (4, 4') et sont espacées l'une de l'autre de sorte que les deux douilles de liaison (4, 4') peuvent être positionnées l'une par rapport à l'autre lors de l'insertion de la tige de liaison (2) immédiatement avant le pivotement de l'instrument d'insertion de tiges (1) au moyen des leviers (1c, 1c').
